# EUROPEAN PATENT SPECIFICATION

(11) **EP 3 145 713 B1**
(45) Date of publication and mention of the grant of the patent: **26.09.2018**
(21) Application number: 15727711.2
(22) Date of filing: 15.05.2015
(51) Int. Cl.: B32B 7/12, A61B 1/00, A61J 1/10, G09F 3/04

(54) **TRANSPORTATION OF MEDICAL INSTRUMENTS**
TRANSPORT VON MEDIZINISCHEN INSTRUMENTEN
TRANSPORT D'INSTRUMENTS MÉDICAUX

(30) Priority: 20.05.2014 GB 201408955
(43) Date of publication of application: 29.03.2017
(73) Proprietor: Tristel PLC, Snailwell, Newmarket CB8 7NY (GB)
(72) Inventor: SWINNEY, Paul, Snailwell Cambridgeshire CB8 7NY (GB); SHABANOVA, Julija, Cambridge Cambridgeshire CB2 9AE (GB); JANSEN, Esther, Cambridge Cambridgeshire CB4 3ES (GB)
(74) Representative: Dummett Copp LLP
(86) International application number: PCT/GB2015/051440
(87) International publication number: WO 2015/177518

(56) References cited:
- EP-A2- 0 134 130
- DE-U1-202004 008 867
- US-A- 5 824 380
- US-A1- 2011 233 079
- US-A1- 2012 294 553

## Description

### FIELD OF THE INVENTION

The present invention relates to a device for the transportation and temporary storage of medical instruments such as endoscopes in a medical facility.

### BACKGROUND TO THE INVENTION

Endoscopes are used for the internal examination of the human or animal body. They are produced in a range of lengths and diameters depending on the intended use. Typically, an endoscope is flexible, and may have an internal channel, or lumen, down which fluids may be directed. Because of the invasive nature of endoscopy, it is necessary that an endoscope be thoroughly cleaned and disinfected after use on a patient before it is used for another procedure. After use, the endoscope undergoes a decontamination procedure involving cleaning, disinfecting and sterilising prior to re-use. WO 2005/107823 discloses a decontamination system which provides cleaning wipes to remove organic deposits, disinfectant/sterilising wipes and sterile rinse wipes to remove disinfectant residues. Each wipe is typically provided in its own sealed sachet which may provide information such as lot or batch number, date of manufacture and expiry date. This information may be transferred to a record book as part of an audit trail to provide a record that an instrument has been properly decontaminated and the date on which this was done.

Each sachet may also optionally be provided with a data carrier such as a bar code or RFID tag, and corresponding data carriers may be provided for the instrument to be decontaminated and for patient and operator details. When carrying out a decontamination procedure, each data carrier is read and a print-out may be produced which provides confirmation that the decontamination procedure has been carried out in accordance with correct procedure, and optionally details such as the instrument decontaminated, the operator, and data specific to a patient. These systems facilitate the provision of proper audit trails to ensure that an instrument is known to have been decontaminated in accordance with procedure.

Ideally, the decontamination procedure is carried out in close proximity to where the endoscope will be used, and preferably immediately before it is to be used. However, such ideal conditions seldom occur. Typically, a decontaminated instrument must be temporarily stored until required, and it may need to be used in a procedure room which is some distance from where decontamination took place.
For transportation of endoscopes before and after use, it has been proposed in WO 2003/034936, to provide a re-usable tray having an endoscope compartment, a single-use disposable tray-liner having an open-faced pouch, and a pouch-closing protective cover. The tray-liner is impermeable to body fluids, and flexible enough that the pouch is able to conform to the contours of the endoscope compartment. When an endoscope is placed in the pouch within the endoscope compartment, the protective cover can be detachably extended across the open face of the pouch from one edge to another so as to enclose and protect the endoscope.
To provide traceability data, an operator may place a ticket carrying the data in the tray, under the tray-liner. A problem with this is that it is necessary to remove the protective cover and the tray-liner to access the traceability data. Movements in the course of this operation generate particles and increase the risk of contamination. An alternative, in which a ticket is placed on the instrument itself, also introduces an undesirable source of potential contamination.
Another transportation system provides two large instrument bags: one for decontaminated endoscopes and the other for contaminated endoscopes. The two bags may be of different colours to allow easy differentiation. However, the action of unfolding the bags is also liable to generate potentially contaminating particles.

US 2011/0233079 discloses a device for the transportation and temporary storage of a medical instrument such as an endoscope, the device comprising: an instrument bag formed of a plastics material substantially impermeable to bodily fluids, the bag having an opening for receiving the instrument; an inner label which has a first portion adhered to an external surface of the bag adjacent to the opening; and an outer label which overlies the inner label and which has a first portion which has a lower surface adhered to at least one of an external surface of the bag and an upper surface of the inner label.

### SUMMARY OF THE INVENTION

According to the present invention there is provided a device as specified in claim 1. Preferred features are specified in the dependent claims.

The invention provides an instrument bag that is easily sealed, ensuring no harmful organisms compromise the sterility of the decontaminated medical device. Contaminated instruments are kept in an enclosed environment, protecting healthcare staff from transmission of potentially harmful pathogens.

The invention also provides for traceability data to be transported along with the medical device, ensuring that patient and instrument records are always linked.

### BRIEF DESCRIPTION OF THE DRAWINGS

The invention will now be further described, by way of example, with reference to the following drawings in which:
Figures 1 and 2 show a device in accordance with an embodiment of the invention, with the inner label respectively concealed and revealed;
Figure 3 is a schematic view of part of the device of Figure 1;
Figures 4 and 5 are plan views showing respectively an outer label and an inner label for use with a device in accordance with an embodiment of the invention;
Figure 6 to 14 illustrate stages in the use of a device in accordance with another embodiment of the invention; and
Figure 15 is a view similar to Figure 6 of a further embodiment of the invention.

### DETAILED DESCRIPTION

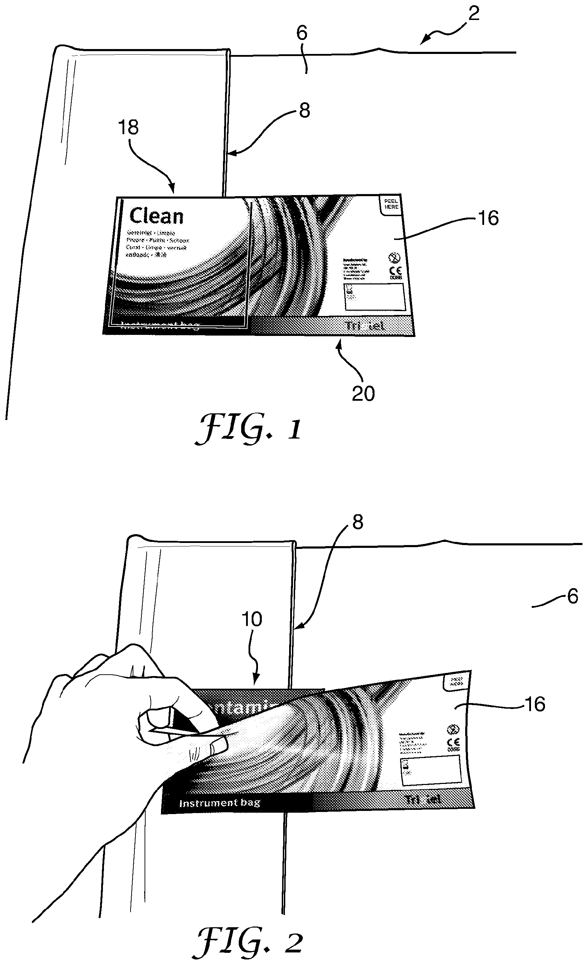

The device 2 shown in Figure 1 provides transportation and temporary storage for a medical instrument such as an endoscope. The device 2 comprises an instrument bag 6 which has an opening 8 for receiving the instrument. The bag 6 is formed from a plastics material which is impermeable to bodily fluids so that a user is protected from potentially harmful pathogens that may be present on a contaminated instrument sealed within the bag 6. The bag 6 has an inner label 10 (Figure 2) and an outer label 16. In this embodiment, the inner label 10 is adhered to an external surface of the bag 6 adjacent to the opening 8. The outer label 16 overlies the inner label 10 and has a first portion 18 which has a lower surface adhered to at least one of an external surface of the bag 6 and an upper surface of the inner label 10. The outer label 16 has a second portion 20 which is disposed beyond the opening 8 and which has a lower surface at least a part of which is adhesive and covered by a removable backing.

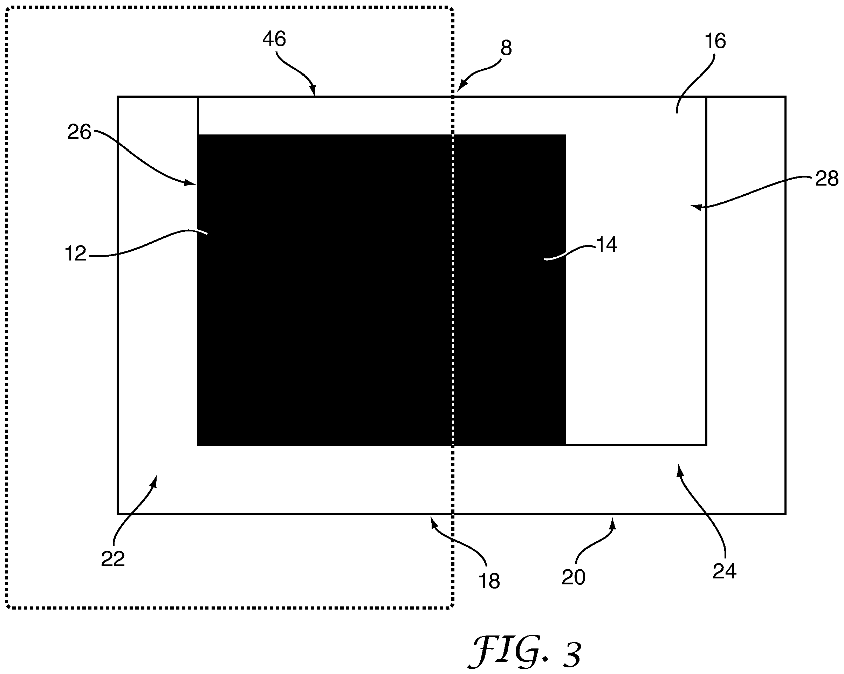

Referring now to Figure 3, a partial x-ray view of an embodiment of the invention shows the inner label 10 comprising a first portion 12 adhered to an external surface of the bag 6 adjacent the opening 8, and a second portion 14 which is disposed beyond the opening 8. The second portion 14 has a lower surface which is adhesive and covered by a removable backing.

The first portion 18 of the outer label 16 in this embodiment includes a first region 26 which does not have an exposed adhesive lower surface and which extends to an edge 46 of the outer label 16, and an area 22 which has an adhesive lower surface which is adhered to the surface beneath and which in this example, is generally L-shaped.

The second portion 20 of the outer label 16 in this embodiment includes a second region 28 which does not have an exposed adhesive lower surface and which extends to an edge 46 of the outer label 16. The lower surface of the second portion 20 of the outer label includes an area 24, which in this example is generally L-shaped. Area 24 has a lower surface which is adhesive and covered by a selectively removable backing. This surface, when exposed, allows a user to seal the bag 6.

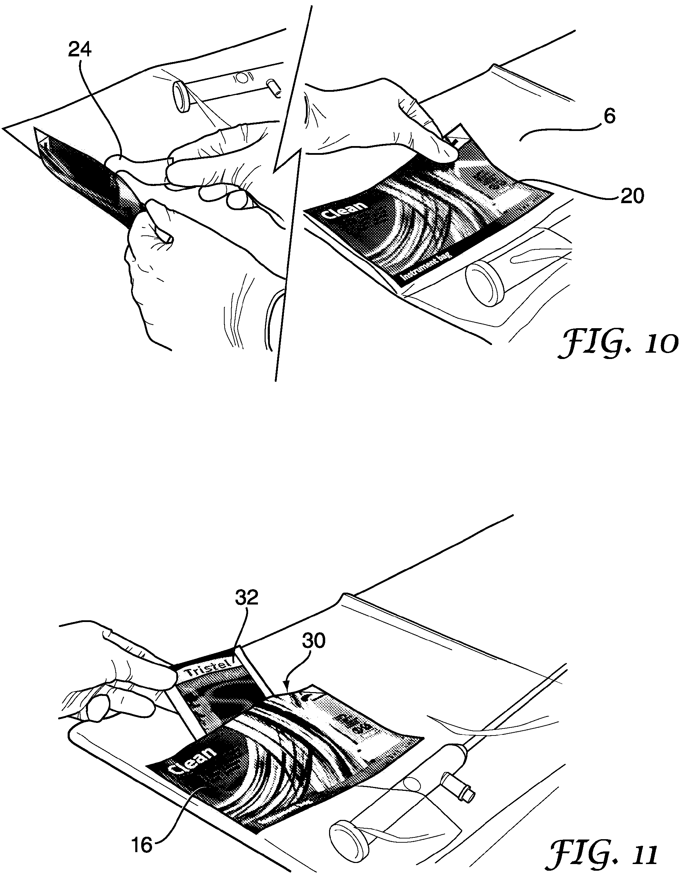

The first region 26 and the second region 28 define a pocket 30 (best shown in Figure 11) for receiving a data carrier 32 such as a disinfectant wipe sachet or a printed ticket carrying traceability information, when the exposed adhesive surface of the second area 24 of the outer label is adhered to an external surface of the bag. It will be appreciated that the pocket could alternatively be provided solely by the first region 26 in an alternative embodiment.

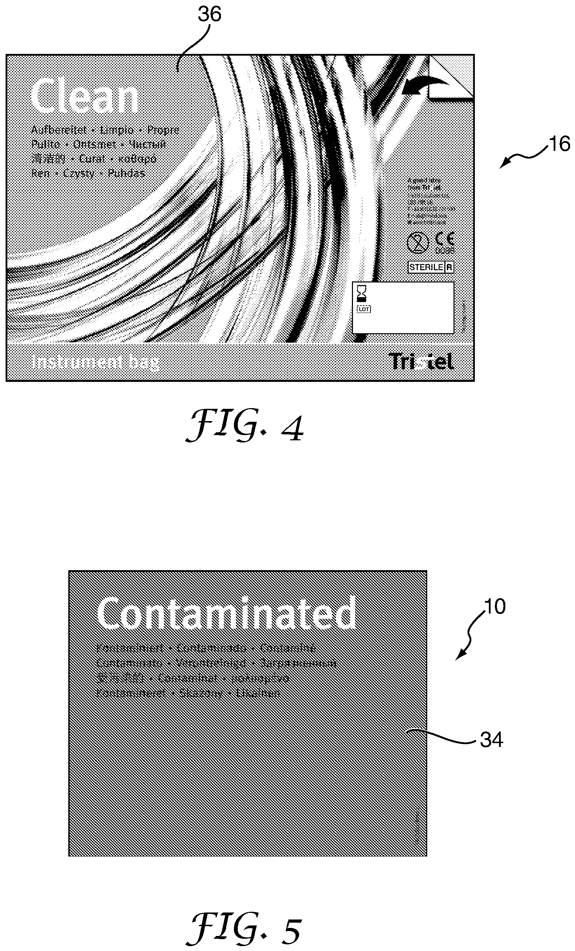

Examples of an outer label 16 and an inner label 10 are shown in Figures 4 and 5 respectively. The outer label 16 has an upper surface 36 which carries information denoting that the contents of the bag are clean. The inner label 10 has an upper surface 34 that carries information denoting that the contents of the bag are contaminated.

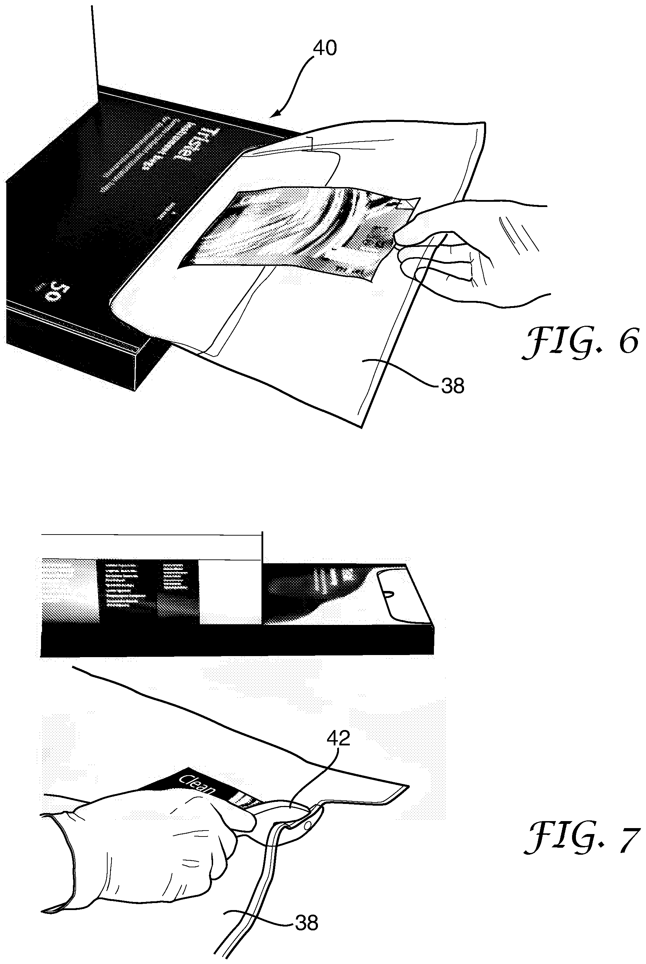

Referring now to Figures 6 to 14, steps in the use of a device in accordance with an embodiment of the invention are as follows. Each instrument bag 6 may be provided in its own outer bag 38 which is formed of a fluid-impervious material and is sealed to prevent ingress of contaminants.

The instrument bags 6 may be sterilised by gamma-irradiation either before or after insertion in the outer bag 38.

The sealed outer bags containing the instrument bags are provided, in this example, in boxes 40.

When a decontaminated instrument is to be transported to a theatre of use, the user removes one outer bag 38 and its contents from the box 40 (Figure 6).

The user cuts the short edge of the outer bag 38 with a cutting implement 42, allowing the contents to be accessed (Figure 7).

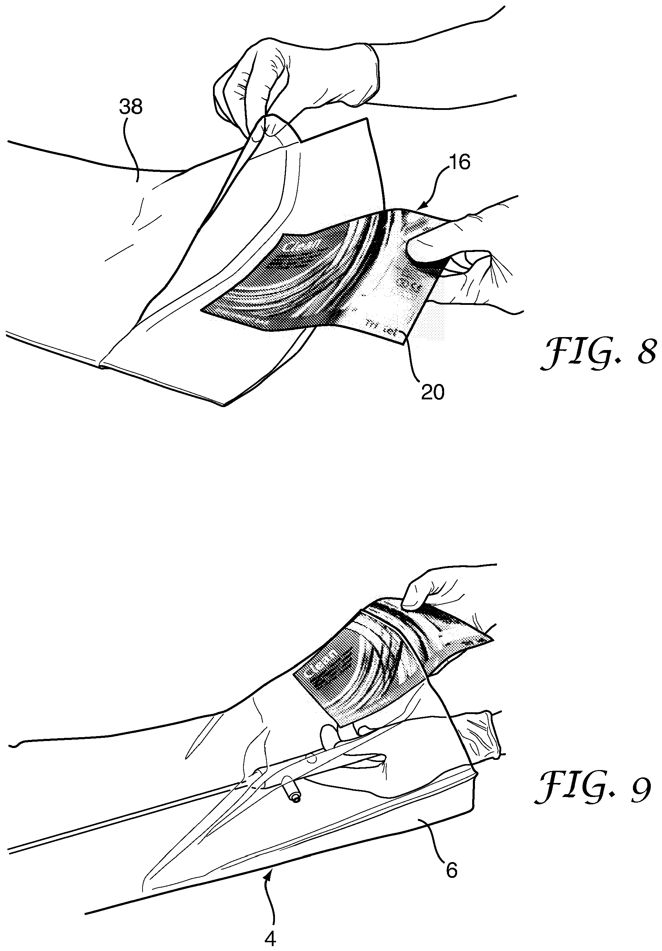

The user removes the instrument bag from the outer bag 38 by pulling the free second portion 20 of the outer label 16 (Figure 8). The outer bag 38 may now be disposed of.

Still holding the second portion of the outer label, the user holds the instrument bag 6 open with minimal contact and inserts the clean medical device 4, which in this example is an endoscope (Figure 9).

The L-shaped backing 24 is peeled off the second portion 20 of the outer label, and the instrument bag is sealed by pressing of the exposed adhesive portion onto an outer surface of the instrument bag (Figure 10).

The user then places a data carrier 32 into the pocket 30. In this example the data carrier is a sachet of the sporicidal wipe used to disinfect the medical device 4 such as is described in WO 2005/107823; it will be understood that other data carriers may be used which will provide appropriate traceability information. Such information may include, but is not limited to, patient notes, instrument records, lot or batch number of the disinfectant used on the instrument, use-by date of the disinfectant. The medical device 4 is now ready for safe transport to the next patient, together with traceability data relating its disinfection (Figure 11).

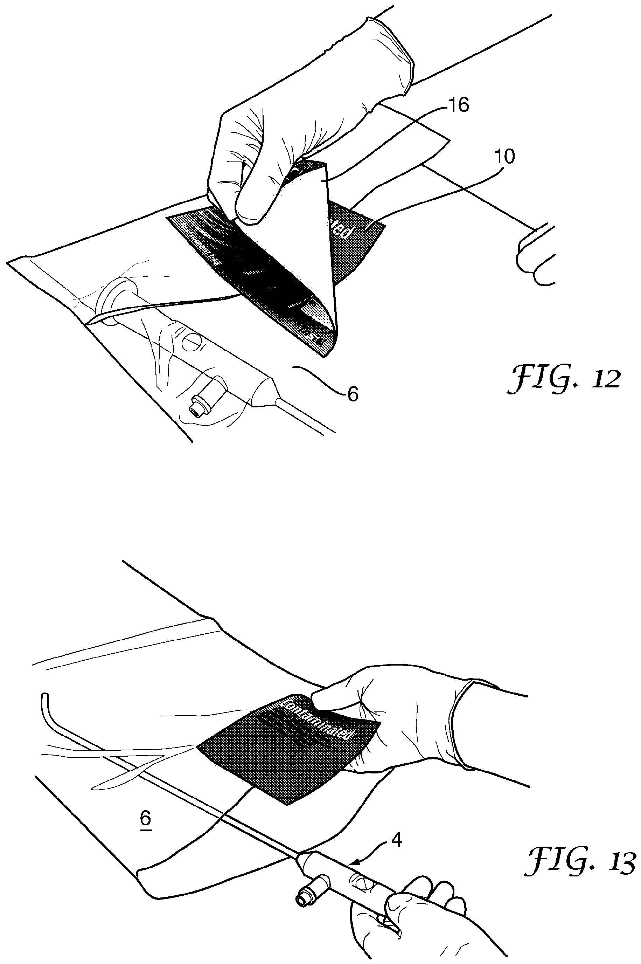

When the instrument is to be used (Figure 12) medical professional sees that the instrument is clean and takes out the data carrier 32. The instrument bag 6 is opened by peeling off the outer label 16, which may then be discarded. The clean seal is now broken and the inner label 10 is revealed (Figure 12). The clean instrument 4 is removed from the instrument bag 6 for use (Figure 13) but the bag 6 is retained.

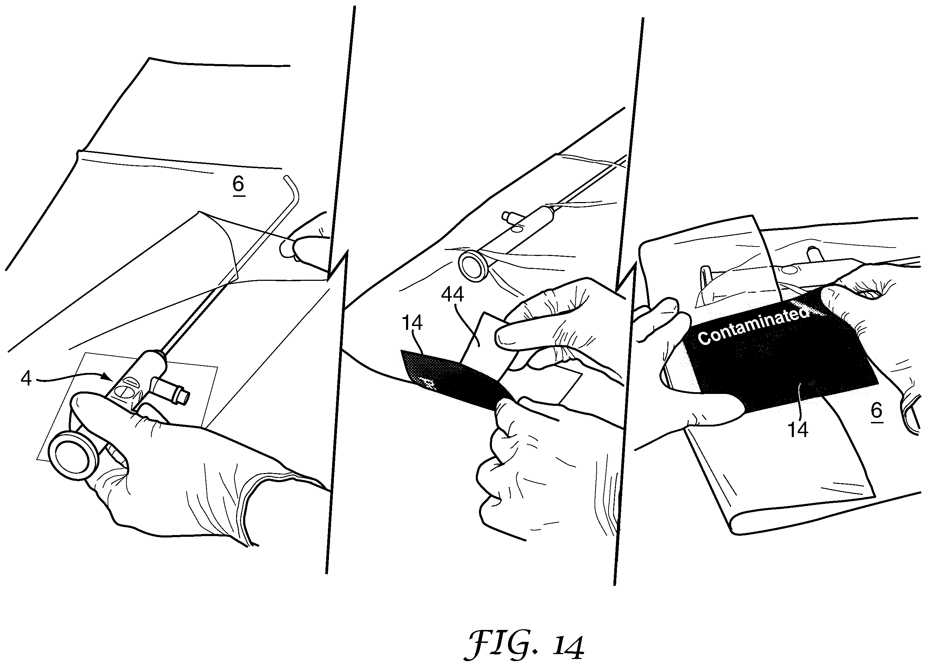

After use, the contaminated instrument 4 is placed back in the instrument bag 6. It is preferred that the opening of the bag is sealed, and in this embodiment, the inner label has a free second portion 14 with an adhesive lower surface and a removable backing 44. The user peels off the backing 44 and reseals the bag 6 with the contaminated instrument 4 within it (Figure 14). The instrument 4 is now ready for safe transport to the decontamination room.

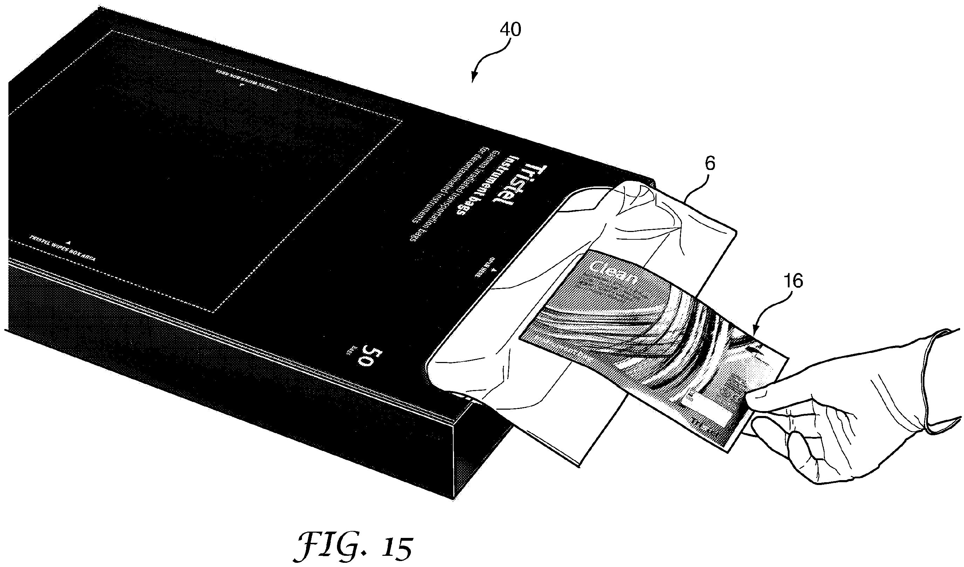

In the embodiment of Figure 15, the optional outer bag is omitted. The instrument bags 6 are sterile (in this embodiment, having been sterilised by gamma-irradiation) and are packed in a box 40. Sterilisation may conveniently be carried out by gamma-irradiation on the devices when packed in the box. A user can remove the devices in turn as required by gripping and pulling on the outer label 16. Further steps are as illustrated with respect to Figures 9-14.

It will be appreciated that various sizes of instrument bag may be used depending on the size of the instruments they are to contain.

The terms "upper" and "lower" and the like are relative and to be construed having regard to the context of the described embodiments.

The articles "a" and "an" are used herein to mean "at least one" where the context permits.

## Claims

1. A device for the transportation and temporary storage of a medical instrument such as an endoscope, the device comprising:
an instrument bag formed of a plastics material substantially impermeable to bodily fluids, the bag having an opening for receiving the instrument;
an inner label which has a first portion adhered to an external surface of the bag adjacent to the opening and has a second portion which is disposed beyond the opening and which has a lower surface which is adhesive and covered by a removable backing; and
an outer label which overlies the inner label and which has a first portion which has a lower surface adhered to at least one of an external surface of the bag and an upper surface of the inner label, and a second portion which is disposed beyond the opening and which has a lower surface at least part of which is adhesive and covered by a removable backing.

2. A device according to claim 1, wherein the lower surface of the first portion of the outer label has a first region which is not adhered to the surface beneath and which extends to an edge of the outer label.

3. A device according to claim 2, wherein the lower surface of the second portion of the outer label includes an area which has a selectively removable backing to provide an exposed adhesive surface and a second region which does not have an exposed adhesive surface and which extends to an edge of the outer label.

4. A device according to claim 3, wherein the first region and the second region together define a pocket for receiving a data carrier when the outer label is adhered to an external surface of the bag.

5. A device according to claim 3 or claim 4, wherein the selectively removable backing is generally L-shaped.

6. A device according to any preceding claim, wherein the adhesive lower surface of the first portion of the outer label is generally L-shaped.

7. A device according to any preceding claim, wherein the inner label has an upper surface which carries information denoting that the contents of the bag are contaminated.

8. A device according to any preceding claim, wherein the outer label has an upper surface which carries information denoting that the contents of the bag are clean.

9. A device according to any preceding claim, further comprising an outer bag which contains the instrument bag, the outer bag being formed of a fluid-impervious material and sealed to prevent ingress of contaminants.

10. A device according to claim 1, wherein the lower surface of the first portion of the outer label has a first region which is not adhered to the surface beneath and which extends to an edge of the outer label and defines a pocket for a data carrier.

## Patentansprüche

1. Vorrichtung zum Transportieren und vorübergehenden Aufbewahren eines medizinischen Instruments, wie zum Beispiel eines Endoskops, wobei die Vorrichtung folgendes umfasst:
einen Instrumentenbeutel, der aus einem Kunststoffmaterial ausgebildet ist, welches im Wesentlichen für Körperflüssigkeiten undurchlässig ist,
wobei der Beutel eine Öffnung zur Aufnahme des Instruments aufweist;
ein inneres Etikett, welches einen ersten Teil aufweist, der an einer Außenfläche des Beutels angrenzend an die Öffnung angeklebt ist, und einen zweiten Teil aufweist, welcher unterhalb der Öffnung angeordnet ist und welcher eine untere Fläche aufweist, die klebend und mit einem entfernbaren Abdeckmaterial abgedeckt ist;
ein äußeres Etikett, welches über dem inneren Etikett liegt und einen ersten Teil aufweist, welcher eine untere Fläche aufweist, die wenigstens an der Außenfläche des Beutels oder an einer oberen Fläche des inneren Etiketts angeklebt ist, und einen zweiten Teil aufweist, welcher unterhalb der Öffnung angeordnet ist und eine untere Fläche aufweist, von welcher wenigstens ein Teil klebend ausgebildet und mit einem entfernbaren Abdeckmaterial abgedeckt ist.

2. Vorrichtung nach Anspruch 1,
bei welcher die untere Fläche des ersten Teils des äußeren Etiketts einen ersten Bereich aufweist, welcher nicht mit der darunter liegenden Fläche verklebt ist und welcher sich bis zu einem Rand des Etiketts erstreckt.

3. Vorrichtung nach Anspruch 2,
bei welcher die untere Fläche des zweiten Teils des äußeren Etiketts eine Fläche beinhaltet, welche ein selektiv zu entfernendes Abdeckmaterial umfasst, um eine freiliegende Klebefläche bereitzustellen, und einen zweiten Bereich beinhaltet, welcher keine freiliegende Klebefläche aufweist und welcher sich bis zu einem Rand des äußeren Etiketts erstreckt.

4. Vorrichtung nach Anspruch 3,
bei welcher der erste Bereich und der zweite Bereich zusammen eine Tasche zur Aufnahme eines Datenträgers definieren, wenn das äußere Etikett an die Außenfläche des Beutels geklebt ist.

5. Vorrichtung nach Anspruch 3 oder Anspruch 4,
bei welcher das selektiv entfernbare Abdeckmaterial grundsätzlich L-förmig ist.

6. Vorrichtung nach einem der vorigen Ansprüche,
bei welcher die klebende untere Fläche des ersten Teils des äußeren Etiketts grundsätzlich L-förmig ist.

7. Vorrichtung nach einem der vorigen Ansprüche,
bei welcher das innere Etikett eine obere Fläche aufweist, welche Informationen trägt, die angeben, dass der Inhalt des Beutels kontaminiert ist.

8. Vorrichtung nach einem der vorigen Ansprüche,
bei welcher das äußere Etikett eine obere Fläche aufweist, welche Informationen trägt, die angeben, dass der Inhalt des Beutels sauber ist.

9. Vorrichtung nach einem der vorigen Ansprüche,
weiter umfassend einen äußeren Beutel, der den Instrumentenbeutel enthält, wobei der äußere Beutel aus einem fluidundurchlässigen Material gebildet und verschlossen ist, um den Eintritt von Verunreinigungen zu verhindern.

10. Vorrichtung nach Anspruch 1,
bei welcher die untere Fläche des ersten Teils des äußeren Etiketts einen ersten Bereich aufweist, welcher nicht an der darunter liegenden Fläche klebt und welcher sich bis zu einem Rand des äußeren Etiketts erstreckt und eine Tasche für den Datenträger definiert.

## Revendications

1. Dispositif pour le transport et le stockage temporaire d'un instrument médical tel qu'un endoscope, le dispositif comprenant :
un sac à instrument formé d'une matière plastique sensiblement imperméable aux fluides corporels, le sac ayant une ouverture pour recevoir l'instrument ;
une étiquette intérieure qui a une première partie amenée à adhérer à une surface externe du sac adjacente à l'ouverture et a une seconde partie qui est disposée au-delà de l'ouverture et qui a une surface inférieure qui est adhésive et recouverte par une protection amovible ; et
une étiquette extérieure qui recouvre l'étiquette intérieure et qui a une première partie qui a une surface inférieure amenée à adhérer à au moins une parmi une surface externe du sac et une surface supérieure de l'étiquette intérieure, et une seconde partie qui est disposée au-delà de l'ouverture et qui a une surface inférieure dont au moins une partie est adhésive et recouverte par une protection amovible.

2. Dispositif selon la revendication 1, dans lequel la surface inférieure de la première partie de l'étiquette extérieure a une première région qui n'est pas amenée à adhérer à la surface au-dessous et qui s'étend jusqu'à un bord de l'étiquette extérieure.

3. Dispositif selon la revendication 2, dans lequel la surface inférieure de la seconde partie de l'étiquette extérieure comprend une région qui a une protection sélectivement amovible pour fournir une surface adhésive exposée et une seconde région qui n'a pas de surface adhésive exposée et qui s'étend jusqu'à un bord de l'étiquette extérieure.

4. Dispositif selon la revendication 3, dans lequel la première région et la seconde région définissent ensemble une poche pour recevoir un support de données lorsque l'étiquette extérieure est amenée à adhérer à une surface externe du sac.

5. Dispositif selon la revendication 3 ou la revendication 4, dans lequel la protection sélectivement amovible est généralement en forme de L.

6. Dispositif selon l'une quelconque des revendications précédentes, dans lequel la surface inférieure adhésive de la première partie de l'étiquette extérieure est généralement en forme de L.

7. Dispositif selon l'une quelconque des revendications précédentes, dans lequel l'étiquette intérieure a une surface supérieure qui porte des informations indiquant que le contenu du sac est contaminé.

8. Dispositif selon l'une quelconque des revendications précédentes, dans lequel l'étiquette extérieure a une surface supérieure qui porte des informations indiquant que le contenu du sac est propre.

9. Dispositif selon l'une quelconque des revendications précédentes, comprenant en outre un sac extérieur qui contient le sac à instrument, le sac extérieur étant formé d'un matériau imperméable aux fluides et scellé pour empêcher la pénétration de contaminants.

10. Dispositif selon la revendication 1, dans lequel la surface inférieure de la première partie de l'étiquette extérieure a une première région qui n'est pas amenée à adhérer à la surface au-dessous et qui s'étend jusqu'à un bord de l'étiquette extérieure et qui définit une poche pour un support de données.
